# EUROPEAN PATENT SPECIFICATION

(11) **EP 3 116 425 B1**
(45) Date of publication and mention of the grant of the patent: **16.12.2020**
(21) Application number: 15761966.9
(22) Date of filing: 13.03.2015
(51) Int. Cl.: A61B 17/80

(54) **PLATES FOR GENERATING, APPLYING AND MAINTAINING COMPRESSION WITHIN A BODY**
PLATTEN ZUR ERZEUGUNG ,ANWENDUNG UND AUFRECHTERHALTUNG VON KOMPRESSION IN EINEM KÖRPER
PLAQUES DE GÉNÉRATION, D'APPLICATION ET DE MAINTIEN DE COMPRESSION À L'INTÉRIEUR D'UN CORPS

(30) Priority: 13.03.2014 US 201461952524 P
(43) Date of publication of application: 18.01.2017
(73) Proprietor: Arthrex, Inc., Naples, FL 34108-1945 (US)
(72) Inventor: PALMER, Matthew, Medford, MA 02155 (US); FONTE, Matthew, Concord, MA 01742 (US); DEVANEY, Robert, Auburndale, MA 02466 (US); NEALON, Kaitlyn, Sommerville, MA 02143 (US)
(74) Representative: Lohr, Jöstingmeier & Partner
(86) International application number: PCT/US2015/020598
(87) International publication number: WO 2015/138995

(56) References cited:
- WO-A2-2005/086708
- FR-A1- 2 626 460
- US-A- 2 580 821
- US-A- 4 905 679
- US-A- 5 246 443
- US-A- 5 660 188
- US-A1- 2008 071 373
- US-A1- 2008 147 125
- US-A1- 2010 036 430
- US-A1- 2011 060 372
- US-A1- 2013 030 437

## Description

### Field Of The Invention

The present invention relates to plates for generating, applying, and maintaining compression to a site in a human or animal body in order to facilitate healing of diseased or damaged tissue. The invention finds particular utility in the field of orthopedics and specifically for reducing fractures and maintaining compression between bone fragments. While the invention has application throughout the body, its utility will be illustrated herein in the context of the repair of fractured or displaced bone tissue, such as during a Calcaneal-Cuboid Arthrodesis, Metatarsal Shortening and/or Distal Radius Fixation. The present invention also finds utility as a cervical compression plate, and/or as other spinal compression plates.

### Background Of The Invention

In the field of orthopedic surgery it is common to rejoin broken bones. The success of the surgical procedure often depends on the ability to reapproximate the bone fragments, the amount of compression achieved between the bone fragments, and the ability to sustain that compression over a period of time. If the surgeon is unable to bring the bone fragments into close contact, a gap will exist between the bone fragments and the bone tissue will need to fill that gap before complete healing can take place. Furthermore, gaps between bone fragments that are too large allow motion to occur between the bone fragments, disrupting the healing tissue and thus slowing the healing process. Optimal healing requires that the bone fragments be in close contact with each other, and for a compressive load to be applied and maintained between the bone fragments. Compressive strain between bone fragments has been found to accelerate the healing process in accordance with Wolf's Law.

Broken bones can be rejoined using plates. These plates are generally formed from a sheet or ribbon of material with a plurality of holes formed therein. The plates are typically manufactured from either stainless steel alloys or titanium alloys. The plates are placed adjacent to a fracture so that the plate spans the fracture line, and then screws are inserted through the holes in the plate and into the bone fragments on either side of the fracture site so as to stabilize the bone fragments relative to one another.

While these plates are designed to stabilize a fracture, they do not always succeed in generating a compressive load between the bone fragments. It is widely reported that the compressive load of plates dissipates rapidly as the bone relaxes and remodels around the screws which hold the plate to the bone.

Thus there exists a clinical need for new and improved compression plates which are able to bring bone fragments into close proximity with each other, generate a compressive load, and maintain that compressive load for a prolonged period of time while healing occurs.

### Summary Of The Invention

Similar plates are known in the prior art for instance from the US 2013030437 application which relates to staples used for fixation of bone and soft tissue of the musculoskeletal system and the methods for their use and more specifically to staples that are caused to change shape through their metallurgic properties and their interaction with mechanical instruments to pull together and compress bone.

A further similar device is described in the prior art document US2010036430. This relates to a snap-off surgical screw configured for threading into a bone of a patient using a driver member, comprising a shaft extension joined to a screw portion via a frangible connection. The frangible connection comprises at least one defect formed through an outer surface of the frangible connection. The defect is configured to promote selective separation of the shaft extension from the screw portion at the defect. A proximal end of the screw portion is preferably provided with a recess, and the frangible connection is preferably positioned in the recess to thereby configure the shaft extension to snap-off from the screw portion below the proximal end of the screw portion.

Another similar device is also known from US 2580821 which relates to appliances for manipulating and holding two sections or fragments of a fractured bone in normal or proper alinement with the fractured ends of the sections in proper contact or abutting relation with each other to facilitate healing or knitting together thereof.

In the US publication US 2008/071373 A1, orthopaedic distraction implants and techniques are disclosed. A tool for handling these implants is not disclosed. A clip and osteosynthesis plate with dynamic compression and self-retention is disclosed in US 5,246,443 A.

A bone fixing device which may be attached by bone screws is disclosed in US 2011/060372 A1.

A bone fracture reduction device and a method of internal fixation of bone fractures is disclosed in US 4, 905,679. The device is comprised of at least two spaced bone affixation plate sections which have a screw hole for receiving a bone screw.

In WO 2005/086708 A2, a flexible anterior cervical plate is disclosed. The plate is designed to flex axially and laterally in response to an axial load. It further has through-holes for bone screws.

In FR 2 626 460 A1, an osteosynthesis plate comprising two sections with through-holes for bone screws is disclosed.

US 2008/147125 A1 discloses an active settling plate and a method of use. It comprises two plate members having a plurality of bone screw holes which are slideably connected together.

The present invention provides a novel compression plate which is able to bring bone fragments into close proximity with each other, generate a compressive load, and maintain that compressive load for a prolonged period of time while healing occurs.

Among other things, the present invention comprises the provision and use of a novel compression plate which is manufactured from a single piece of shape memory material (e.g., a material capable of exhibiting superelasticity and/or a temperature-induced shape change). The shape memory material may be a metal alloy (e.g., Nitinol) or a polymer (e.g., appropriately processed PEEK). The novel compression plate is designed to reduce fractures and generate and maintain more uniform compression between the cortical bone and cancellous bone of the bone fragments so as to aid in fracture healing.

In one form of the invention, the novel compression plate comprises two opposing regions joined together by a pair of elastic bridge members, and an opening in each of the two opposing regions for receiving screws. In the un-restrained state, the two elastic bridge members are bowed outwardly. Prior to implantation, the two elastic bridge members can be reversibly strained inwardly so that the two elastic bridge members are nearly parallel (i.e., the two elastic bridge members are stretched laterally inwardly). A delivery device may be used to accomplish this straining of the compression plate and to hold the compression plate in this strained state prior to implantation. Upon implantation, the constraint on the two elastic bridge members is removed, whereupon the two elastic bridge members attempt to return to their original unrestrained state, thereby generating a compressive load and maintaining that compressive load for a prolonged period of time while healing occurs.

In one preferred form of the invention, there is provided apparatus for providing compression within a body, said apparatus comprising:
a compression plate comprising first and second opposing regions connected together by at least one elastic bridge member, wherein said at least one elastic bridge member has a non-linear configuration when it is in its unbiased condition but is capable of being elastically deformed to a more linear configuration upon the application of force to said at least one elastic bridge member, and at least one opening formed in each of said first and second opposing regions, wherein said at least one opening in each of said first and second opposing regions is configured to receive a fastener;
such that said openings in said first and second opposing regions are separated by a first distance when said at least one elastic bridge member is in its said non-linear configuration, and said openings in said first and second opposing regions are separated by a second distance when said at least one elastic bridge member is in its said more linear configuration, and further wherein said second distance is greater than said first distance.

In another preferred form of the invention, there is provided a compression plate for providing compression to first and second bone fragments across a fracture line, said compression plate comprising:
first and second opposing regions connected together by at least one elastic bridge member, wherein said at least one elastic bridge member has a non-linear configuration when it is in its unbiased condition but is capable of being elastically deformed to a more linear configuration upon the application of force to said at least one elastic bridge member; and
at least one opening formed in each of said first and second opposing regions, wherein said at least one opening in each of said first and second opposing regions is configured to receive a fastener;
such that when said at least one elastic bridge member is elastically strained into its said more linear configuration, and said compression plate is positioned against bone so that one of said openings is positioned over the first bone fragement and one of said openings is positioned over the second bone fragment, with said at least one elastic bridge member spanning the fracture line, and when a fastener is passed through one opening and into the first bone fragment and another fastener is passed through the other opening and into the second bone fragment, and the strain on said at least one elastic bridge member is thereafter released, said compression plate will apply a compressive force across the fracture line.

In another preferred form of the invention, there is provided a method for applying compression to first and second bone fragments across a fracture line, said method comprising:
providing apparatus for providing compression within a body, said apparatus comprising:
   a compression plate comprising first and second opposing regions connected together by at least one elastic bridge member, wherein said at least one elastic bridge member has a non-linear configuration when it is in its unbiased condition but is capable of being elastically deformed to a more linear configuration upon the application of force to said at least one elastic bridge member, and at least one opening formed in each of said first and second opposing regions, wherein said at least one opening in each of said first and second opposing regions is configured to receive a fastener;
   such that said openings in said first and second opposing regions are separated by a first distance when said at least one elastic bridge member is in its said non-linear configuration, and said openings in said first and second opposing regions are separated by a second distance when said at least one elastic bridge member is in its said more linear configuration, and further wherein said second distance is greater than said first distance;
elastically straining said at least one elastic bridge member into its said more linear configuration, and positioning said compression plate against the first and second bone fragments so that one of said openings is positioned over the first bone fragement and one of said openings is positioned over the second bone fragment, with said at least one elastic bridge member spanning the fracture line, and passing a fastener through one opening and into the first bone fragment and passing another fastener through the other opening and into the second bone fragment; and
releasing the strain on said at least one elastic bridge member so that said compression plate will apply a compressive force across the fracture line.

In another preferred form of the invention, there is provided a compression plate comprising:
first and second opposing regions connected together by at least one bridge member; and
at least one opening formed in each of said first and second opposing regions, wherein said at least one opening in each of said first and second opposing regions is configured to receive a fastener;
wherein said at least one bridge member is formed out of austenite but capable of forming stress-induced martensite;
and further wherein at least one of said opposing regions is formed out of fully annealed Nitinol or martensitic Nitinol with an austenite start temperature greater than body temperature.

In another preferred form of the invention, there is provided apparatus for providing distraction within a body, said apparatus comprising:
a distraction plate comprising first and second opposing regions connected together by at least one elastic bridge member, wherein said at least one elastic bridge member has a more linear configuration when it is in its unbiased condition but is capable of being elastically deformed to a less linear configuration upon the application of force to said at least one elastic bridge member, and at least one opening formed in each of said first and second opposing regions, wherein said at least one opening in each of said first and second opposing regions is configured to receive a fastener;
such that said openings in said first and second opposing regions are separated by a first distance when said at least one elastic bridge member is in its said more linear configuration, and said openings in said first and second opposing regions are separated by a second distance when said at least one elastic bridge member is in its said less linear configuration, and further wherein said first distance is greater than said second distance.

In another preferred form of the invention, there is provided a method for applying distraction to first and second bone segments, said method comprising:
providing apparatus for providing distraction to first and second bone segments, said apparatus comprising:
   a distraction plate comprising first and second opposing regions connected together by at least one elastic bridge member, wherein said at least one elastic bridge member has a more linear configuration when it is in its unbiased condition but is capable of being elastically deformed to a less linear configuration upon the application of force to said at least one elastic bridge member, and at least one opening formed in each of said first and second opposing regions, wherein said at least one opening in each of said first and second opposing regions is configured to receive a fastener;
   such that said openings in said first and second opposing regions are separated by a first distance when said at least one elastic bridge member is in its said more linear configuration, and said openings in said first and second opposing regions are separated by a second distance when said at least one elastic bridge member is in its said less linear configuration, and further wherein said first distance is greater than said second distance;
elastically straining said at least one elastic bridge member into its said less linear configuration, and positioning said distraction plate against the first and second bone segments so that one of said openings is positioned over the first bone segment and one of said openings is positioned over the second bone segment, and passing a fastener through one opening and into the first bone segment and passing another fastener through the other opening and into the second bone segment; and
releasing the strain on said at least one elastic bridge member so that said distraction plate will apply a distraction force to the first and second bone segments.

### Brief Description Of The Drawings

These and other objects and features of the present invention will be more fully disclosed or rendered obvious by the following detailed description of the preferred embodiments of the invention, which is to be considered together with the accompanying drawings wherein like numbers refer to like parts, and further wherein:
Fig. 1 is a schematic view of a novel compression plate formed in accordance with the present invention, wherein the compression plate comprises a pair of bridge members which are capable of being elastically strained, two openings for receiving screws, and further wherein the compression plate is shown in its unstrained condition;
Fig. 2 is a schematic view of the novel compression plate shown in Fig. 1, wherein the pair of elastic bridge members of the compression plate have been elastically strained to a near-parallel condition;
Fig. 3 is a schematic view showing how the elastically strained compression plate of Fig. 2 will foreshorten when the strain on the compression plate is removed;
Fig. 4 is a schematic view of another novel compression plate formed in accordance with the present invention, wherein the compression plate comprises a pair of bridge members which are capable of being elastically strained, wherein each of the opposing regions of the compression plate comprises two openings for receiving screws, and further wherein the compression plate is shown in its unstrained condition;
Fig. 5 is a schematic view of the novel compression plate shown in Fig. 4, wherein the two elastic bridge members of the compression plate have been elastically strained to a near-parallel condition;
Fig. 6 is a schematic view showing how the elastically strained compression plate of Fig. 5 will foreshorten when the strain on the compression plate is removed;
Fig. 7 is a schematic view of another novel compression plate formed in accordance with the present invention, wherein the compression plate comprises a single "S-shaped" bridge member which is capable of being elastically strained, two openings for receiving screws, and further wherein the compression plate is shown in its unstrained (i.e., unstretched) condition;
Fig. 8 is a schematic view of the novel compression plate shown in Fig. 7, wherein the single "S-shaped" elastic bridge member of the compression plate has been elastically strained (i.e., longitudinally stretched);
Fig. 9 is a schematic view showing how the elastically strained compression plate of Fig. 7 will foreshorten when the strain on the compression plate is removed;
Figs. 10A-10F are schematic views showing other novel compression plates formed in accordance with the present invention - these compression plates may be used when a more tailored anatomical fixation is required (e.g., for Cranio-Maxillofacial fracture fixation);
Fig. 11 is a schematic view of a screw system which can be used to attach the novel compression plate of the present invention to bone - in this embodiment, the screw is a snap-off screw that detaches from the driver when the screw has been fully seated in the bone;
Fig. 12 is a schematic view showing snap-off screws being used to attach the novel compression plate to the bone;
Fig. 13 is a schematic view showing a delivery device which may be used with the novel compression plate shown in Fig. 4 to elastically strain (i.e., stretch) the two elastic bridge members of the compression plate so as to make them parallel (or at least more parallel than their unconstrained state);
Fig. 14 is a schematic view showing how the delivery device shown in Fig. 13 engages the novel compression plate shown in Fig. 4;
Fig. 15 is a schematic cross-sectional view of the delivery device showing in Fig. 13;
Fig. 16 is a schematic view of an alternative delivery device which may be used with the novel compression plate shown in Fig. 2;
Figs. 17 and 18 are schematic views showing how the novel compression plate shown in Figs. 4 and 5 may be used to provide compression across a bone fracture;
Fig. 19 is a schematic view of another novel compression plate formed in accordance with the present invention, wherein the compression plate is contoured so as to allow for multiple compression plates to be assembled together into a custom plate configuration, and further wherein the two elastic bridge members of the compression plate can be elastically strained to a near-parallel condition;
Fig. 20 is a schematic view of a multi-member compression plate configuration made up of a plurality of the compression plates shown in Fig. 19;
Fig. 21 is a schematic view of the lap joint which is created when two of the compression plates of Fig. 19 are assembled together;
Fig. 22 is a schematic view of another novel compression plate formed in accordance with the present invention, wherein areas of the compression plate (e.g., the two opposing regions of the compression plate) have been selectively treated so as to allow for plastic deformation to occur in those areas, and elastic strain to occur in other areas of the compression plate (e.g., the two elastic bridge members of the compression plate);
Figs. 23-25 are schematic views showing plastic threaded inserts disposed between the openings of the compression plate and the fixation screws which are used to hold the compression plate to bone; and
Fig. 26 is a schematic view of a novel distraction plate formed in accordance with the present invention.

### Detailed Description Of The Preferred Embodiments

### Novel Compression Plate Comprising Elastic Bridge Members

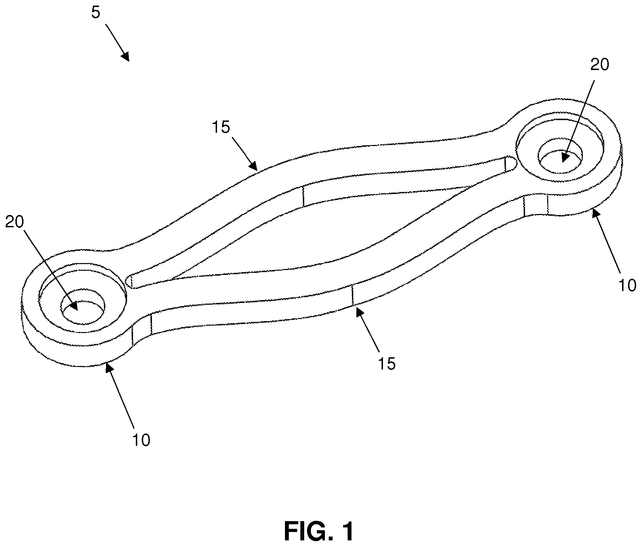

Looking first at Fig. 1, there is shown a novel compression plate 5 which is able to bring bone fragments into close proximity with each other, generate compressive load across the fracture line (i.e., between the cortical bone and the cancellous bone of the bone fragments), and maintain that compressive load for a prolonged period of time while healing occurs.

Novel compression plate 5 is preferably a structure manufactured from a single piece of shape memory material (e.g., a material capable of exhibiting superelasticity and/or a temperature-induced shape change). The shape memory material may be a metal alloy (e.g., Nitinol) or a polymer (e.g., appropriately processed PEEK). Compression plate 5 is designed to reduce fractures and generate and maintain compression between bone fragments in order to aid in fracture healing. Compression plate 5 generally comprises two opposing regions 10 joined together by a pair of elastic bridge members 15, and at least one opening 20 formed in each of the two opposing regions 10 for receiving fixation screws. Openings 20 may have a countersunk feature (e.g., a bore-counterbore configuration) so as to allow the heads of the fixation screws to sit substantially flat with the top surface of compression plate 5. Additionally, openings 20 may be threaded so as to allow for positive engagement between the openings and the threaded fixation screws. In the un-restrained state, elastic bridge members 15 are bowed outwardly, such as in the manner shown in Fig. 1.

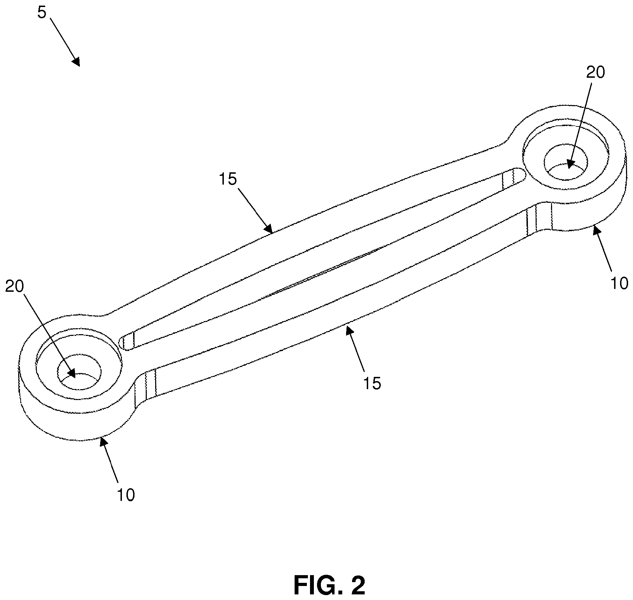

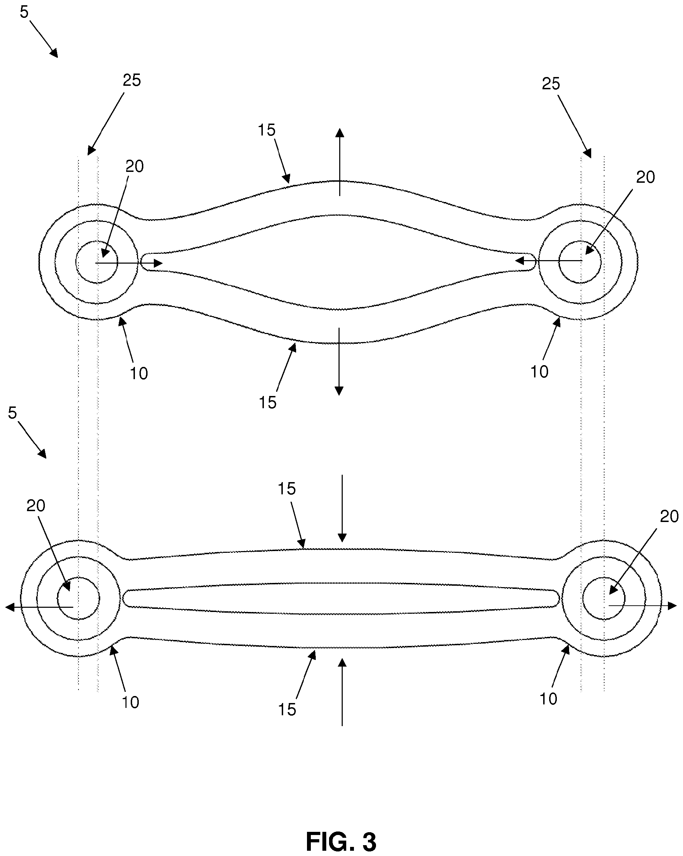

Prior to implantation, elastic bridge members 15 of compression plate 5 can be reversibly strained inwardly (i.e., bent laterally inwardly), thus increasing the distance 25 (Fig. 3) between opposing regions 10 (and hence openings 20) of compression plate 5 (Fig. 2). Note that where compression plate 5 is formed out of Nitinol, elastic deformations of up to approximately 8% are achievable. A delivery device (see below) can be used to strain elastic bridge members 15 so as to bring elastic bridge members 15 to a substantially parallel state.

During implantation, the strained (i.e., elongated) compression plate 5 is positioned against the bone fragments and secured to those bone fragments by passing fixation screws through openings 20 and into the bone fragments.

Removal of the induced strain on compression plate 5 (provided by the delivery device, see below) results in compression plate 5 attempting to return to its original un-restrained state (Fig. 3), thereby generating a compressive load on the bone (i.e., through elastic bridge members 15, openings 20 and fixation screws extending through openings 20) and maintaining that compressive load on the bone for a prolonged period of time while healing occurs.

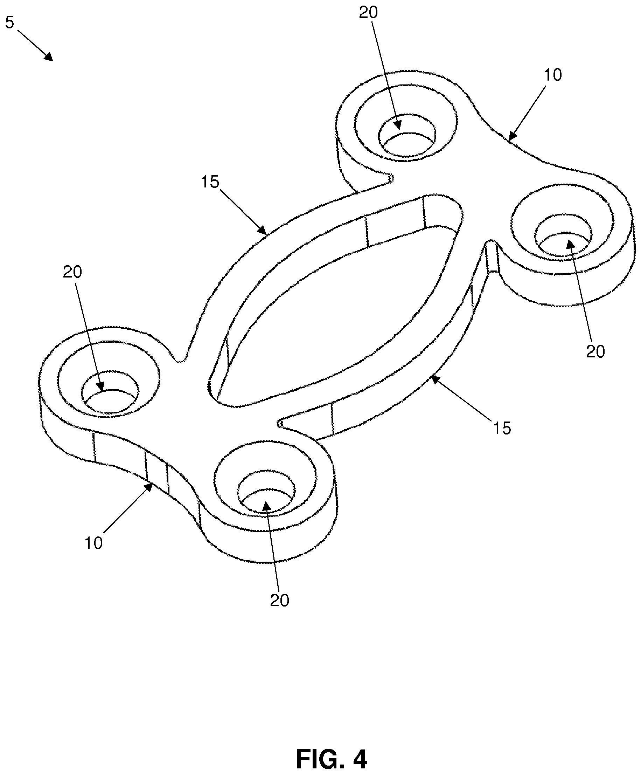

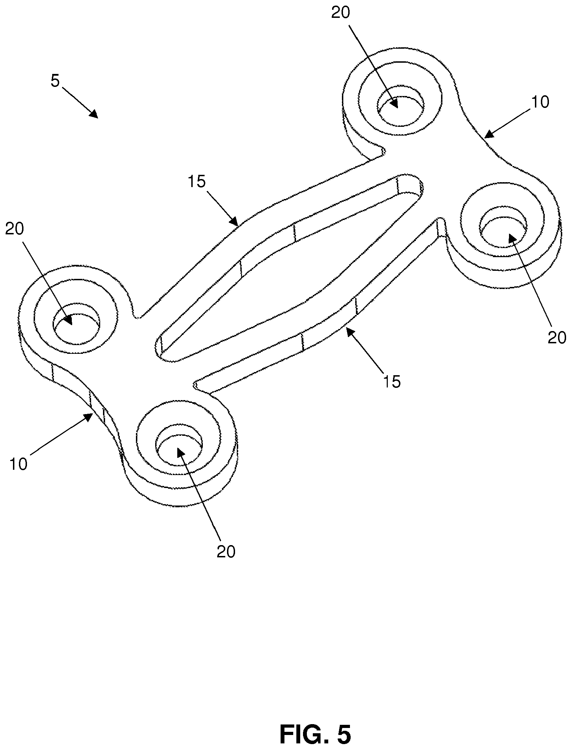

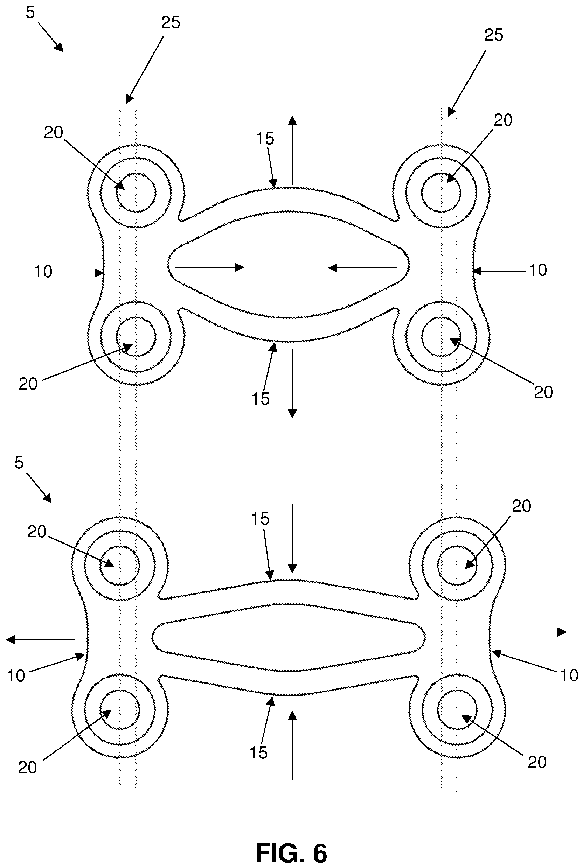

Figs. 4-6 show another compression plate 5 which is generally similar to the compression plate 5 shown in Figs. 1-3, except that the opposing regions 10 of the compression plate shown in Figs. 4-6 each has two openings 20 for receiving fixation screws.

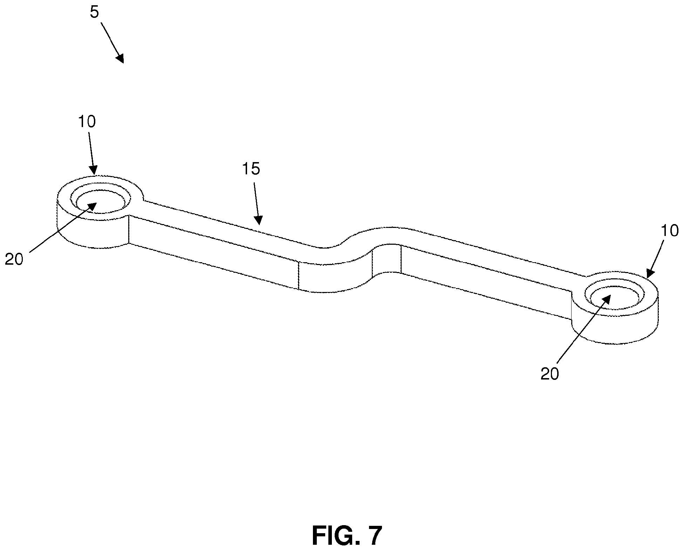

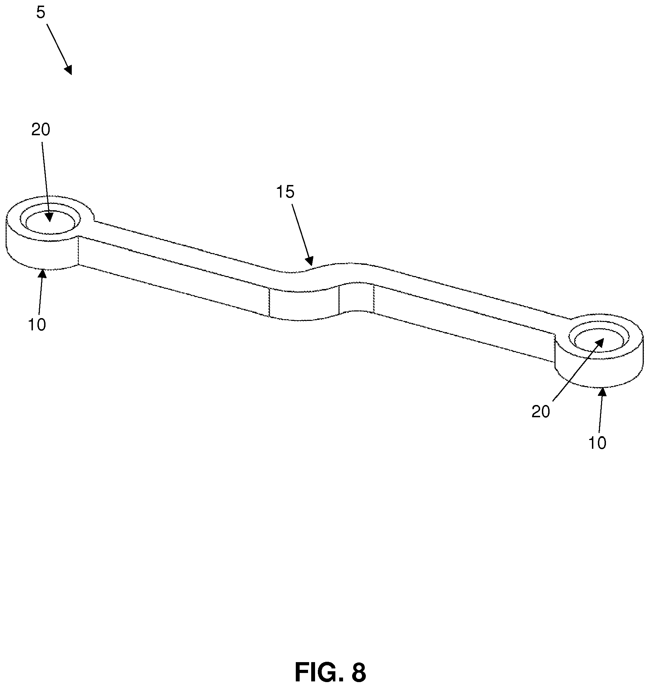

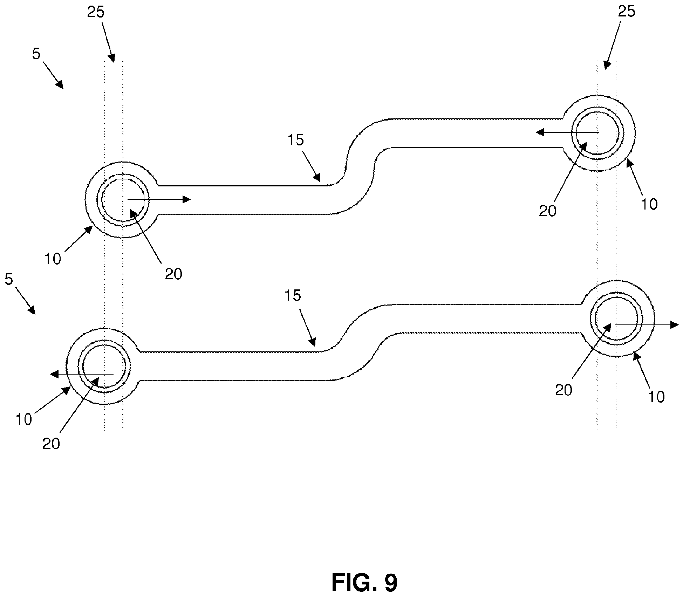

Figs. 7-9 show another novel compression plate 5 also formed in accordance with the present invention. More particularly, the novel compression plate 5 of Figs. 7-9 is generally similar to the compression plate 5 shown in Figs. 1-3, except that the compression plate 5 of Figs. 7-9 has only one elastic bridge member 15, and that elastic bridge member 15 has an "S" configuration. It will be appreciated that the "S" configuration of elastic bridge member 15 of Figs. 7-9 allows the elastic bridge member to be strained so as to move openings 20 further apart, and thereafter relaxed so as to cause a foreshortening of the distance between openings 20. As a result, when compression plate 5 of Figs. 7-9 is strained so as to move openings 20 further apart, and fixation screws are thereafter passed through openings 20 of the strained compression plate and advanced into bone, and the strain on elastic bridge member 15 is thereafter released, the compression plate and fixation screws will apply a compressive force to the bone.

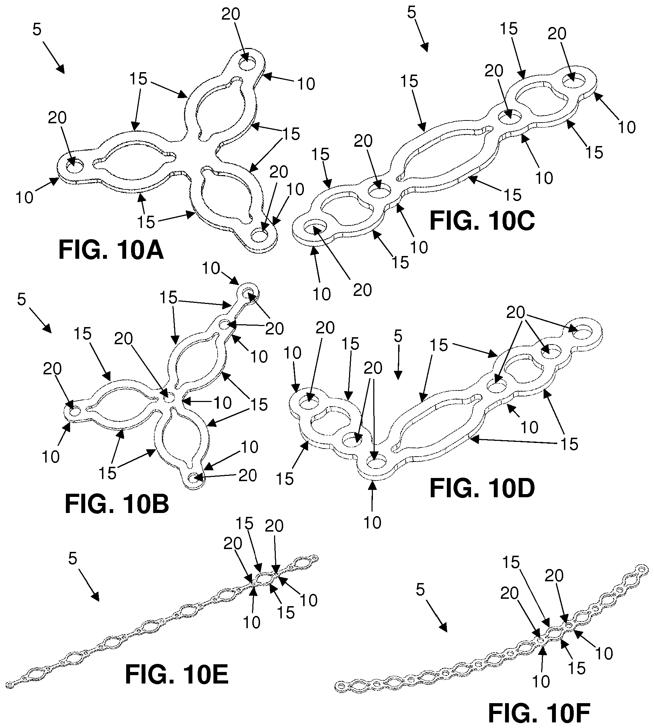

Figs. 10A-10F are schematic views showing additional novel compression plates 5 also formed in accordance with the present invention - these compression plates may be used when more tailored anatomical fixation is required (e.g., for Cranio-Maxillofacial fracture fixation).

Thus it should be appreciated that compression plate 5 may be formed in a variety of linear and non-linear configurations, and may include two or more opposing regions 10 and one or more elastic bridge members 15. Thus, for example, in one form of the invention, compression plate 5 may comprise a linear configuration having two opposing regions 10 connected together by one or more elastic bridge members 15. In another form of the invention, compression plate 5 may comprise a linear configuration having three or more opposing regions 10, each pair of opposing regions being connected together by one or more elastic bridge members 15. In still another form of the invention, compression plate 5 may comprise a non-linear configuration (e.g., a curved configuration) having two opposing regions 10 connected together by one or more elastic bridge members 15. In still another form of the invention, compression plate 5 may comprise a non-linear configuration (e.g., a triangular configuration, a L-shaped configuration, etc.) having three or more opposing regions 10, each pair of opposing regions being connected together by one or more elastic bridge members 15. It will be appreciated that compression plate 5 may also comprise still other configurations, e.g., square, circular, etc., all of which are considered to be within the scope of the present invention.

It will be appreciated that the fixation screws used with compression plate 5 may be conventional fixation screws of the sort well known in the field of fracture fixation.

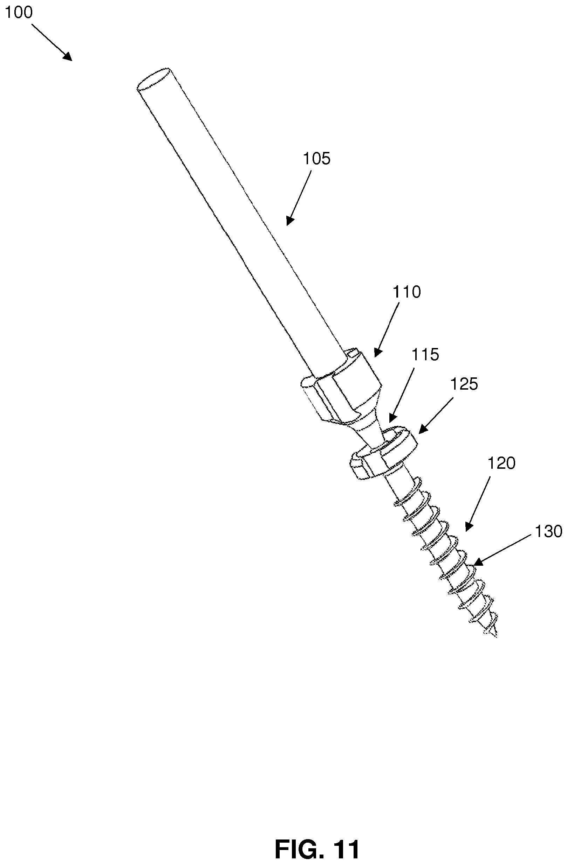

By way of example but not limitation, and looking now at Fig. 11, there is shown a snap-off screw system 100 of the sort well known in the art. This snap-off screw system can be used to secure compression plate 5 to bone. The snap-off screw system 100 has a shaft 105 to engage a rotary driver (not shown), a drive head 110 to engage a screw driver (not shown), a preferred shear location 115, and a fixation screw 120 provided with a screw head 125 and screw threads 130. Screw head 125 may have external threads (not shown) to engage mating threads on opening 20 of compression plate 5, so that fixation screw 120 may function as a locking screw.

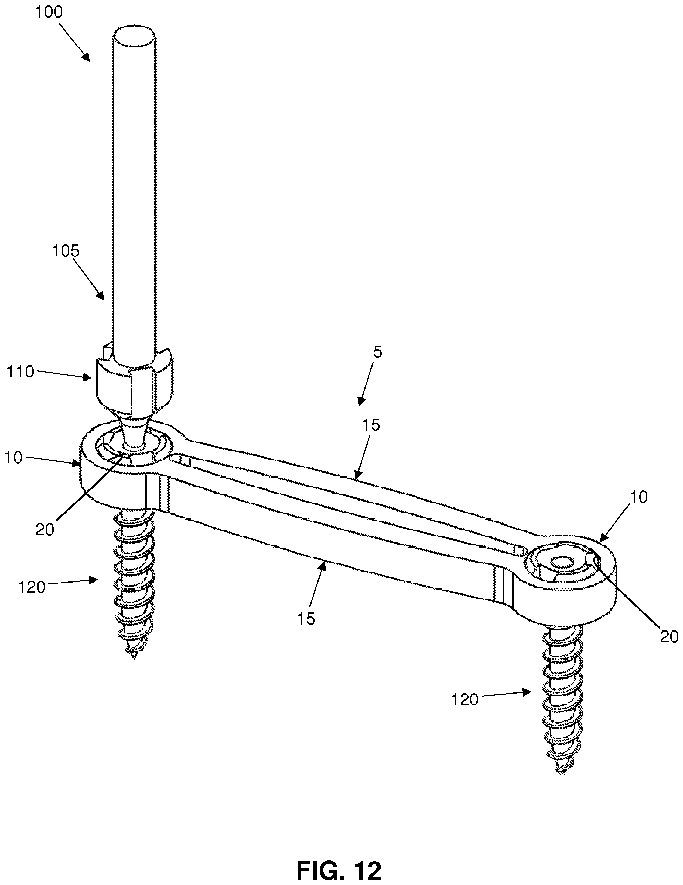

Fig. 12 shows compression plate 5 with snap-off screw system 100 being used to deploy fixation screws 120 through openings 20 and into bone. Note that in Fig. 12, compression plate 5 is shown with its elastic bridge members 35 strained inwardly, although the delivery device (which bends elastic bridge members 15 inwardly) has been omitted for clarity of illustration.

It will be appreciated that various delivery devices may be used to deploy compression plate 5 in the body.

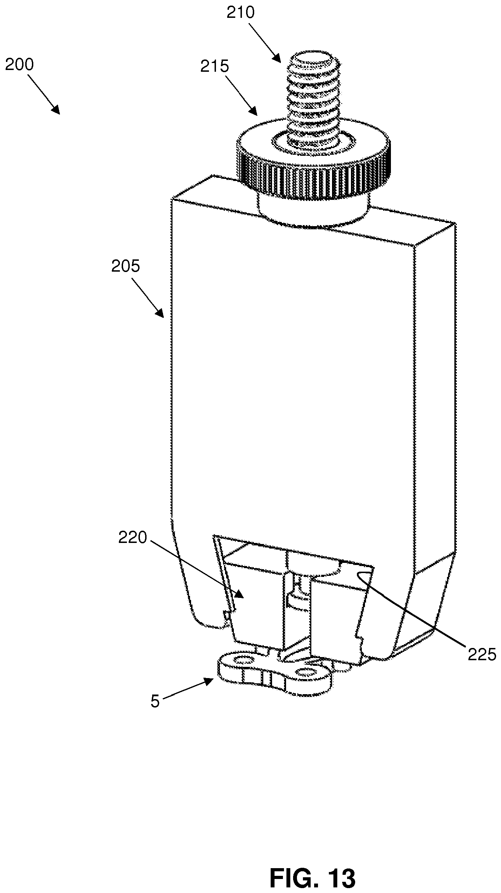

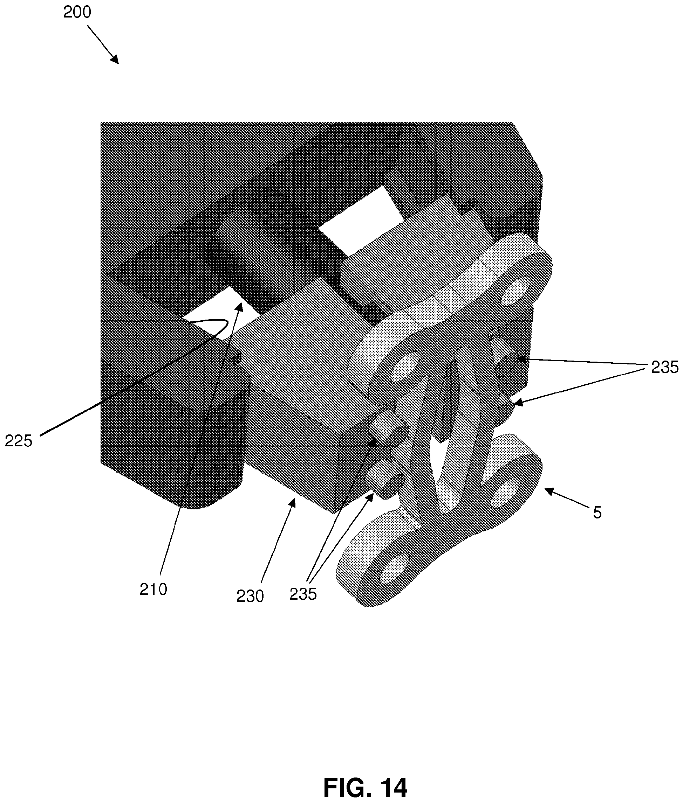

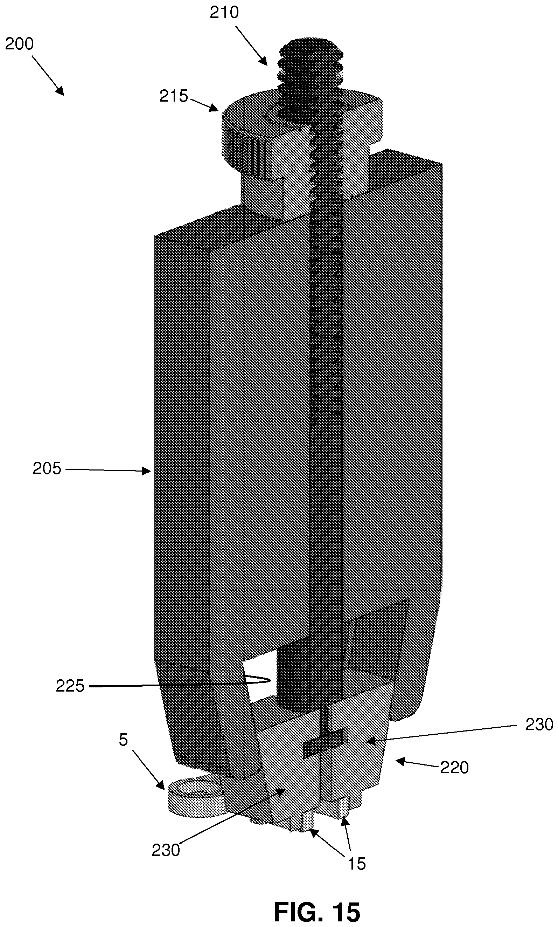

By way of example but not limitation, and looking now at Figs. 13-15, there is shown an exemplary delivery device 200 which may be used to strain (i.e., compress) elastic bridge members 15. Delivery device 200 generally comprises an outer body 205, a central screw 210, a nut 215 and a wedge assembly 220. Plate 5 is engaged by wedge assembly 220 which is movably disposed in a wedge-shaped recess 225 formed in outer body 205. Wedge assembly 220 comprises two individual wedges 230 that ride in wedge-shaped recess 225 (formed in outer body 205) and engage screw 210. The distal end of each individual wedge 230 has two pins 235 that engage elastic bridge members 15 of compression plates 5. When screw 210 is a conventional right-handed thread, loosening nut 215 causes wedge assembly 220 to move downward in wedge-shaped recess 225 of outer body 205, thereby reducing the distance between pins 235. This causes elastic bridge members 15 of compression plate 5 to elastically deform to a near-parallel (or at least to a more parallel) condition. Tightening nut 215 causes wedge assembly 220 to move upward in wedge-shaped recess 225 of outer body 205, thereby increasing the distance between pins 235. This releases the strain on elastic bridge members 15 of compression plate 5, thereby allowing the elastic bridge members 15 to return to their original, outwardly-bowed shape.

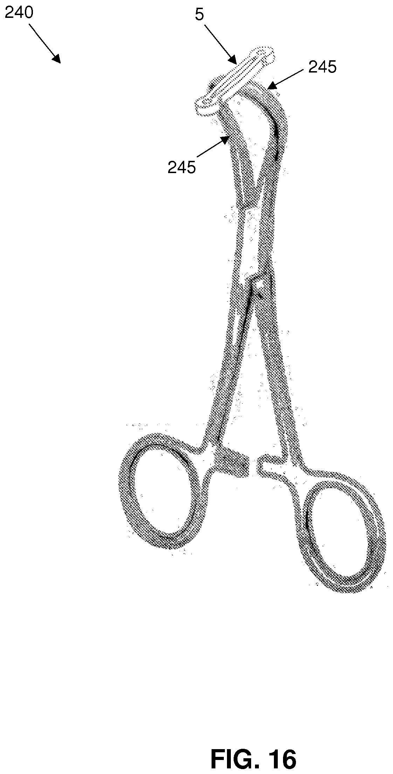

Fig. 16 shows another delivery device 240 which may be used to deploy compression plate 5. The delivery device 240 shown in Fig. 16 is essentially hemostat pliers, with the working tips 245 of the hemostat pliers applying the strain on elastic bridge members 15 of compression plate 5.

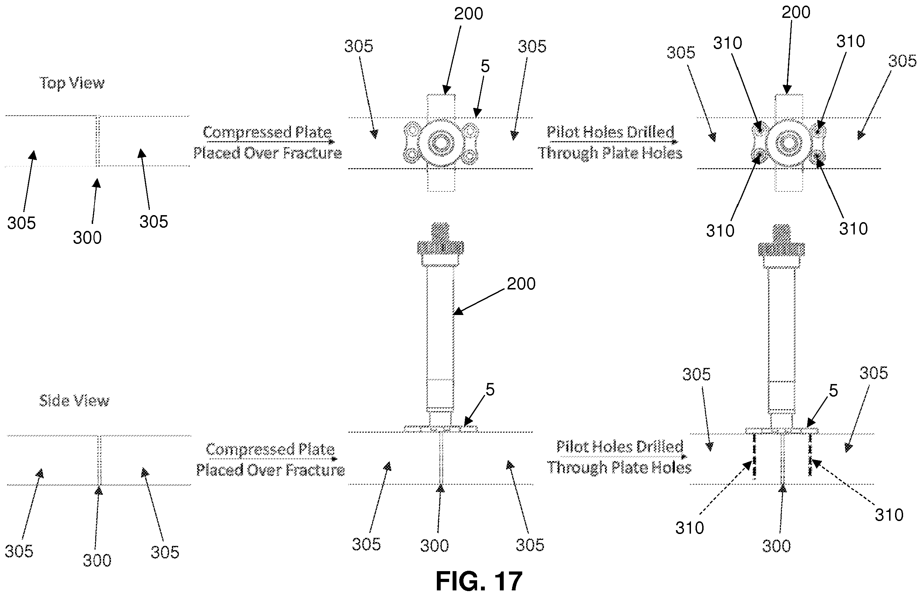

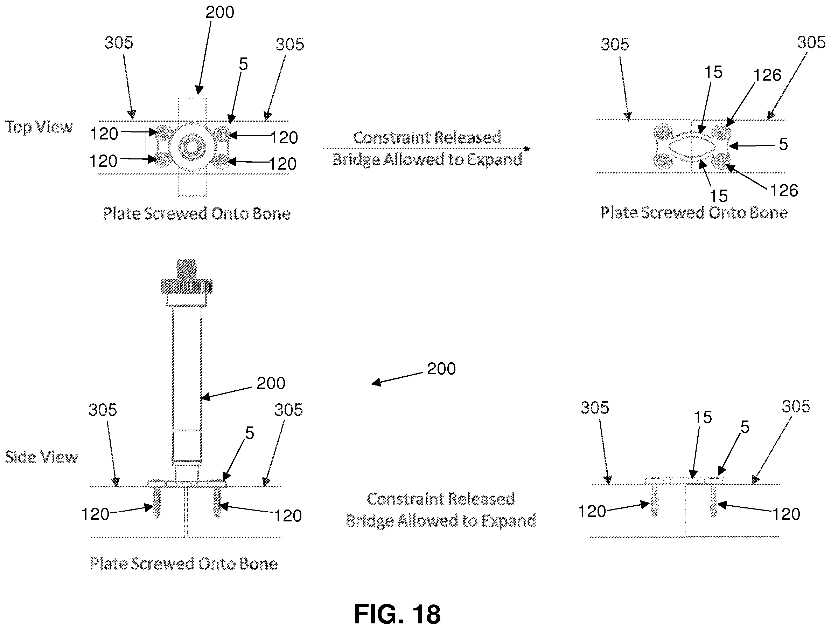

Now next at Fig. 17 and 18, there is shown one preferred method (which is not part of the invention) for treating a fracture 300 formed between two bone fragments 305 using novel compression plate 5.

First, compression plate 5 is loaded onto the delivery device (e.g., delivery device 200 discussed above) and elastic bridge members 15 are compressed so that they are near-parallel (or at least more parallel). The constrained compression plate 5 is then placed over the fracture line 300 and holes 310 are drilled through openings 20. Screws (e.g., snap-off screws 120 of snap-off screw system 100) are installed (i.e., they are advanced through openings 20 and into bone fragments 305), thereby fixing compression plate 5 to the bone fragments 305. The constraining delivery device (e.g., delivery device 200) is then removed and the compression plate 5 is allowed to attempt to regain its original unconstrained shape, thereby generating and maintaining compression across the fracture site.

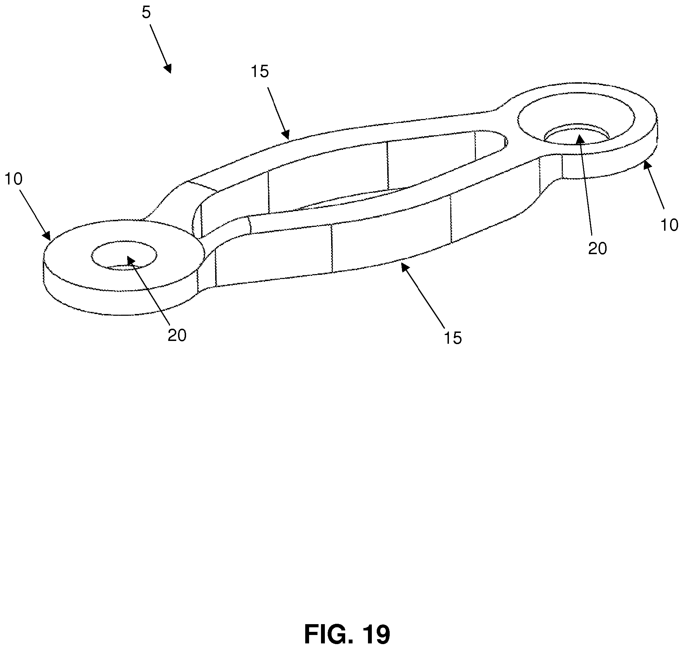

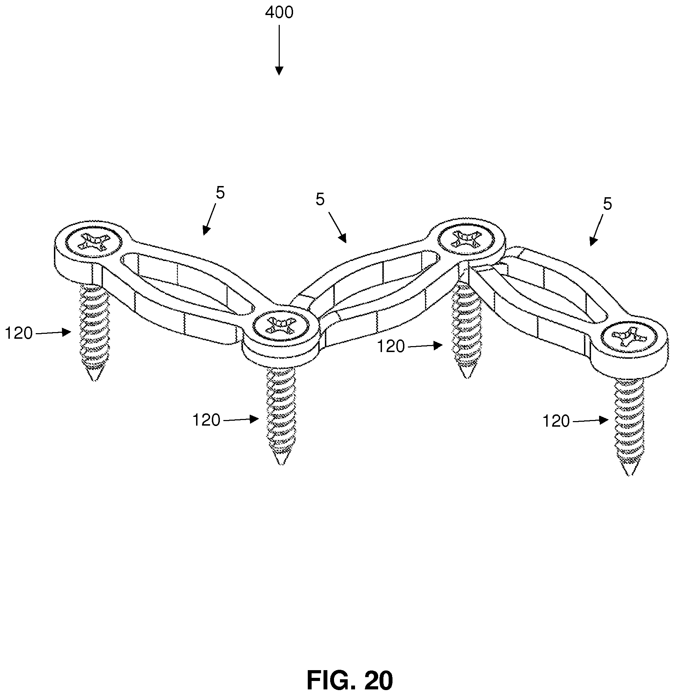

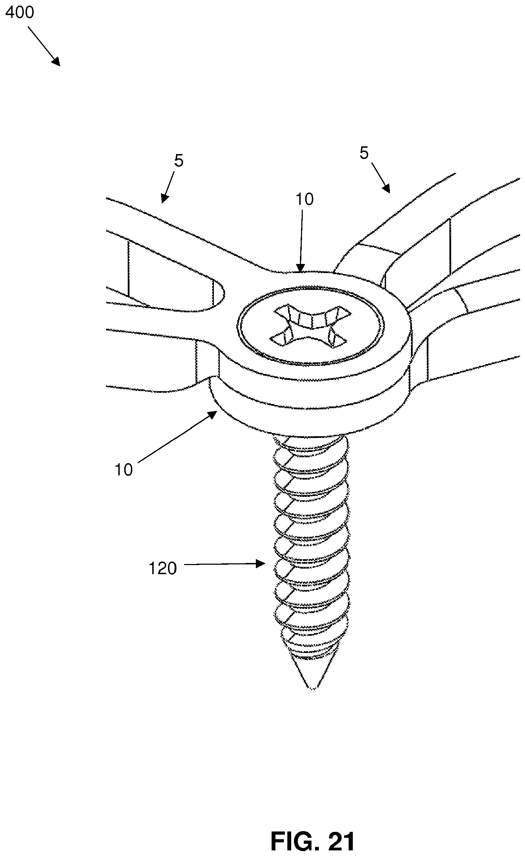

Figs. 19-21 show another novel compression plate 5 formed in accordance with the present invention. More particularly, in this form of the invention, compression plate 5 is configured so as to allow multiple compression plates 5 to be assembled together as a modular plating system 400. If desired, one or both of the two opposing regions 10 of each compression plate 5 may be formed with a height equal to one-half of the height of the two elastic bridge members 15 and, if desired, an opposing region 10 may be aligned with the top surface of the elastic bridge members 15, or the opposing region 10 may be aligned with the bottom surface of the elastic bridge member 15. In this way, adjacent regions 10 can be combined so as to form a lap joint which receives a fixation screw 120.

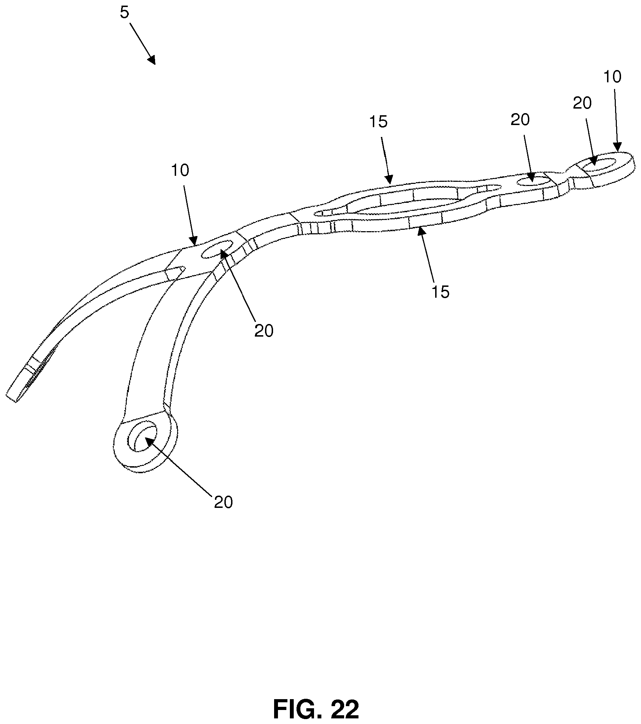

Fig. 22 shows a novel compression plate 5 that has had areas of the compression plate selectively processed (e.g., heat treated) so as to create plastic areas (e.g., opposing regions 10) which can be bent to take a set. This construction allows the compression plate to be contourable to the patient's anatomy (using the areas of plastic deformation) while also generating and maintaining compression across fracture sites (using the areas of elastic deformation). In one preferred form of the invention, compression plate 5 is formed out of Nitinol; the plastic areas of compression plate 5 (e.g., opposing regions 10) are formed out of fully annealed Nitinol or martensitic Nitinol with an austenite start temperature greater than body temperature; and the elastic areas of compression plate 5 (e.g., elastic bridge members 15) are formed out of austenitic Nitinol but capable of forming stress-induced martensite.

Note that compression plate 5 is configured so that the force which is generated as compression plate 5 reconfigures (i.e., as elastic bridge members 15 return outwardly) is less than the "tear through" force of the bone which receives screws 120, i.e., compression plate 5 is specifically engineered so as to not "tear through" the bone tissue when attempting to reconfigure to its original foreshortened configuration. The compressive forces of compression plate 5 can be controlled by (i) modulating the material properties of the compression plate, and/or (ii) varying the geometry of the compression plate.

The percentage of cold work in the shape memory material forming compression plate 5 affects the compressive force generated by the reconfiguring compression plate. As the percentage of cold work increases, the compression force declines. A Nitinol compression plate should, preferably, have between about 15% and 55% cold work to control the recovery force of the compression plate; however, other degrees of cold work may be used, and/or the material may not be cold worked at all.

Another material property that affects the plate's compression force is the temperature differential between the body that the compression plate will be implanted into (assumed to be 37°C, which is the temperature of a human body) and the austenite finish temperature of the shape memory material forming compression plate 5. A smaller temperature differential between the two will result in the compression plate generating a smaller compressive load; conversely, a larger temperature differential between the two will result in the compression plate generating a larger compressive load. When the compression plate is made out Nitinol, the compression plate should, preferably, have an austenite finish temperature of greater than about -10°C, resulting in a temperature differential of about 47°C when the compression plate is implanted (assuming that the compression plate is implanted in a human body).

Plate geometry also affects the compression forces which are generated by the reconfiguring plate. More particularly, the cross-sectional area of elastic bridge members 15 affects the compression forces generated by the reconfiguring plate. As the cross-sectional areas increase, so do the compression forces that the reconfiguring plate will generate. It should be appreciated that the force generated as an elastic bridge member 15 attempts to recover from the constrained linear configuration (Fig. 2) to the bowed outward configuration (Fig. 1) is less than the force which would be generated were the plate to be constructed with linear elastic bridge members that are stretched longitudinally in tension. A plate with linear elastic bridge members that are stretched longitudinally in tension may recover with a force that exceeds the pull-out force in bone.

In one preferred form of the invention, compression plate 5 and delivery device 200 are provided in the form of a sterilized kit. The kit may include additional instruments to aid in the implantation of the compression plate (e.g., k-wire, drill bit, plate size guide, screws, etc.). In one preferred form of the invention, compression plate 5 is provided with an associated delivery device (e.g., delivery device 200) in a sterile package, with elastic bridge members 15 being pre-constrained (e.g., so that elastic bridge members 15 are substantially straight, or at least more straight than when the elastic bridge members are in their unconstrained condition) in the sterile package by the delivery device (e.g., delivery device 200).

### Test Data

Static Elastic Bending: 25mm interaxis width compression plates were tested. A 4-point bending load was applied on a tensile testing machine at a rate of 25.4mm/min. Bending stiffness was calculated as the initial slope of load vs. displacement. The novel compression plate 5 had comparable Bending Strengths (16.33N/mm vs. 15.86N/mm *p*=*0.818*) to conventional fracture fixation plates.

Compression Force: Greater interfragmentary compression has been found to enhance the healing of fractured bones. The compression plates of the present invention utilize the unique superelastic properties of Nitinol to enhance and sustain compression across the fracture plane. The compressive force generated by the novel compression plates of the present invention generate and maintain 180N of compression over nearly 1mm of bone resorption.

### Use Of Plastic Threaded Inserts Between The Openings Of The Compression Plate And The Fixation Screws

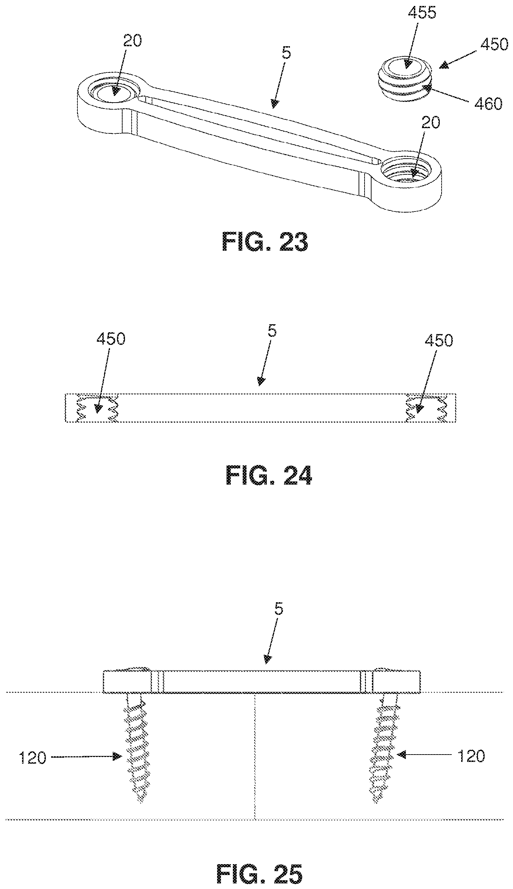

If desired, and looking now at Figs. 23-25, a plastic threaded insert 450 (with a central hole 455 and external threads 460) can be inserted into mating threaded holes 20 on compression plate 5. Threaded plastic inserts 450 can accept a fixation screw (e.g., a fixation screw 120) with appropriate locking threads on the head of the fixation screw. The threads on the head of the fixation screw are able to cut threads into plastic threaded insert 450, locking the fixation screw to compression plate 5 via the intervening plastic threaded insert 450. Note that the fixation screws can be inserted on an angle (e.g., up to 15 degrees) off the center axis of threaded holes 20, allowing for polyaxial fixation of compression plate 5 .

Additionally, it should be appreciated that plastic threaded insert 450 insulates the metallic fixation screw from compression plate 5, thereby limiting galvanic corrosion between the compression plate 5 and the metallic fixation screws.

### Use Of Other Types Of Fasteners With The Novel Compression Plate

In the foregoing disclosure, compression plate 5 is secured to bone fragments using threaded fixation screws. However, if desired, other types of fasteners may also be used to secure compression plate 5 to bone fragments. By way of example but not limitation, pins may be used to secure compression plate 5 to bone fragments.

### Applying Distraction To Bone Using A Novel Distraction Plate

In the foregoing disclosure, compression plate 5 has been discussed in the context of providing compression across a fracture. However, it should also be appreciated that, if desired, the apparatus can be modified so as to provide a distraction force to bone (e.g., to separate two bones or bone fragments, or to induce bone growth in a single bone, etc.).

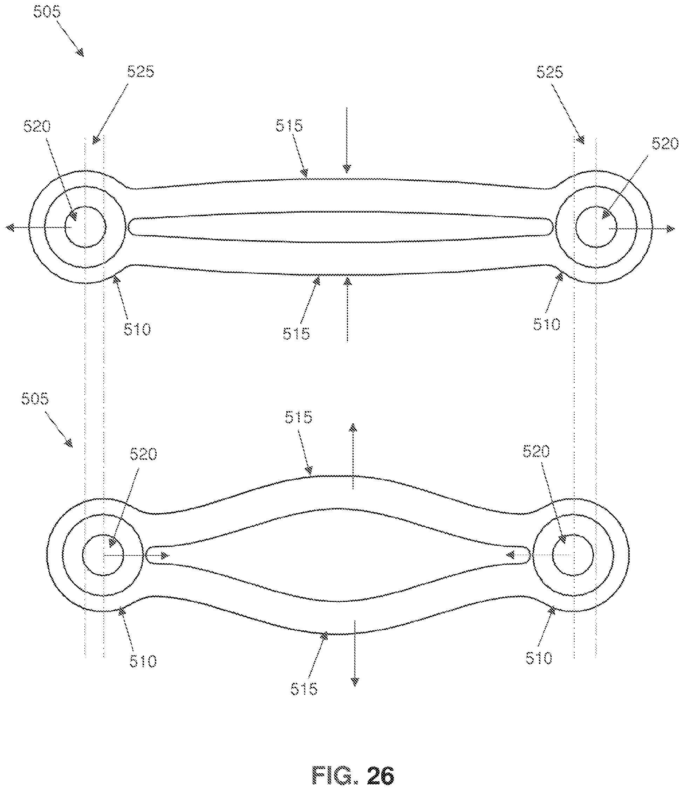

By way of example but not limitation, and looking now at Fig. 26, there is shown a novel distraction plate 505 formed in accordance with the present invention. Distraction plate 505 generally comprises two opposing regions 510 joined together by a pair of elastic bridge members 515, and at least one opening 520 formed in each of the two opposing regions 510 for receiving fixation screws. Openings 520 may have a countersunk feature (e.g., a bore-counterbore configuration) so as to allow the heads of the fixation screws to sit substantially flat with the top surface of distraction plate 505. Additionally, openings 520 may be threaded so as to allow for positive engagement between the openings and the threaded fixation screws. In the un-restrained state, elastic bridge members 515 are linearly aligned, such as in the manner shown in Fig. 26. Note that this is the inverse of the configuration of compression plate 5, where elastic bridge members 15 are bowed outwardly when in their un-restrained state.

Prior to implantation, elastic bridge members 515 of distraction plate 505 can be reversibly strained outwardly (i.e., forced outwardly), thus decreasing the distance 525 between opposing regions 510 (and hence openings 520) of distraction plate 505. A delivery device can be used to strain elastic bridge members 515 so as to force elastic bridge members 515 outwardly, e.g., a delivery device generally similar to the delivery device 200, or the delivery device 240, discussed above, except that the delivery device is configured to force elastic bridge members 515 apart when appropriate.

During implantation, the strained (i.e., the forcibly foreshortened) distraction plate 505 is secured to bone by passing fixation screws through openings 520 and into the bone.

Removal of the induced strain on distraction plate 505 (provided by the aforementioned delivery device) results in distraction plate 505 attempting to return to its original un-restrained state (i.e., with elastic bridge members 515 linearly aligned), thereby generating a distraction load on the bone to which distraction plate 505 is secured (i.e., through elastic bridge members 515, openings 520 and fixation screws extending through openings 520), and maintaining that distraction load on the bone for a prolonged period of time while healing occurs.

### Modifications Of The Preferred Embodiments

It should be understood that many additional changes in the details, materials, steps and arrangements of parts, which have been herein described and illustrated in order to explain the nature of the present invention, may be made by those skilled in the art while still remaining within the principles and scope of the invention.

## Claims

1. An apparatus for providing compression within a body, said apparatus comprising:
a compression plate (5) mounted to a delivery device (200), the compression plate (5) having a center line comprising first and second opposing regions (10) connected together by at least one elastic bridge member (15), **characterized in that**
said at least one elastic bridge member (15) includes a center, wherein said at least one elastic bridge member (15) has a non-linear configuration and said center of said at least one elastic bridge member (15) is bowed outwardly relative to said center line when the at least one elastic bridge member (15) is in its unbiased condition but is capable of being elastically deformed to a more linear configuration upon the application of force to said at least one elastic bridge member (15), and at least one opening (20) formed in each of said first and second opposing regions (10), wherein said at least one opening (20) in each of said first and second opposing regions (10) is configured to receive a fastener (120);
such that said openings (20) in said first and second opposing regions (10) are separated by a first distance when said at least one elastic bridge member (15) is in its said non-linear configuration, and said openings (20) in said first and second opposing regions (10) are separated by a second distance when said at least one elastic bridge member (15) is in its said more linear configuration, and further wherein said second distance is greater than said first distance; and
the delivery device is elastically straining the two elastic bridge members of the compression plate so as to make them at least more parallel than their unconstrained state.

2. An apparatus according to claim 1 wherein said compression plate (5) comprises two elastic bridge members (15), wherein said two elastic bridge members (15) are disposed on either side of said center line, and further wherein said two elastic bridge members (15) are bowed outwardly relative to said center line when said two elastic bridge members (15) are in their unbiased condition.

3. An apparatus according to claim 1 wherein said at least one elastic bridge member (15) has an "S" configuration in its unbiased condition.

4. An apparatus according to claim 1 wherein each of said first and second opposing regions (10) comprises a plurality of openings (20).

5. An apparatus according to any one of the preceding claims wherein said compression plate (5) is formed out of a shape memory material, wherein said shape memory material comprises Nitinol.

6. An apparatus according to claim 1 wherein a portion of said compression plate (5) is plastically deformable, wherein at least one of said first and second opposing regions (10) is plastically deformable.

7. An apparatus according to claim 6 wherein said portion is fully annealed Nitinol or martensitic Nitinol with an austenite start temperature greater than body temperature, wherein said at least one bridge member (15) is formed out of austenite but capable of forming stress-induced martensite.

8. An apparatus according to any one of the preceding claims wherein said more linear configuration is substantially straight.

9. An apparatus according to claim 1 wherein said compression plate (5) is mounted to the delivery device (200) which may be used to reconfigure said at least one elastic bridge member (15) from its said non-linear configuration to its said more linear configuration, wherein said delivery device (200) is user-adjustable to set the extent to which said at least one bridge member (15) is reconfigured.

10. A sterilized kit comprising an apparatus according to claim 9 wherein said compression plate (5) and said delivery device (200) are packaged as the sterilized kit, wherein said compression plate (5) and said delivery device (200) are packaged as the sterilized kit while said delivery device (200) is holding said at least one elastic bridge member (15) in its said more linear configuration.

11. An apparatus according to claim 1 further comprising at least one fastener (120) disposed in an opening, optionally wherein said at least one fastener (120) comprises a fastening screw, and further wherein said at least one opening comprises a screw thread, and further wherein said fastening screw (120) comprises a threaded head (130) for engaging said screw thread.

12. An apparatus according to any one of the preceding claims further comprising a third opposing region (10) connected by at least one additional elastic bridge member (15) to at least one of said first and second opposing regions (10), wherein said at least one additional elastic bridge member (15) has a non-linear configuration when it is in its unbiased condition but is capable of being elastically deformed to a more linear configuration upon the application of force to said at least one additional elastic bridge member (15), and at least one additional opening (20) formed in said third opposing region (10), wherein said at least one additional opening (20) is configured to receive a fastener (120).

13. An apparatus according to claim 1, wherein the compression plate (5) is configured to provide compression to first and second bone fragments across a fracture line,
wherein when said at least one elastic bridge member (15) is elastically strained into said more linear configuration, said compression plate (5) is positioned against bone so that one of said openings (20) is positioned over the first bone fragment and another of said openings (20) is positioned over the second bone fragment, with said at least one elastic bridge member (15) spanning the fracture line,
wherein a fastener (120) passes through one opening (20) and into the first bone fragment and another fastener (120) passes through the other opening (20) and into the second bone fragment, and
wherein said compression plate (5) applies a compressive force across the fracture line when the strain on said at least one elastic bridge member (15) is released.

14. An apparatus according to claim 1, wherein said at least one bridge member (15) is formed out of austenite but capable of forming stress-induced martensite;
and further wherein at least one of said opposing regions (10) is formed out of fully annealed Nitinol or martensitic Nitinol with an austenite start temperature greater than body temperature.

15. An apparatus for providing distraction within a body, said apparatus comprising:
a distraction plate (505) comprising first and second opposing regions (510) connected together by at least one elastic bridge member (515), wherein said at least one elastic bridge member (515) has a linear configuration when it is in its unbiased condition but is capable of being elastically deformed to a less linear configuration upon the application of force to said at least one elastic bridge member (515), and at least one opening (520) formed in each of said first and second opposing regions (510),
wherein said at least one opening (520) in each of said first and second opposing regions (510) is configured to receive a fastener;
such that said openings (520) in said first and second opposing regions (510) are separated by a first distance when said at least one elastic bridge member (515) is in its said linear configuration, and said openings (520) in said first and second opposing regions (510) are separated by a second distance when said at least one elastic bridge member (515) is in its said less linear configuration, and further wherein said first distance is greater than said second distance, and
the distraction plate (505) is mounted to a delivery device (200), the delivery device is elastically straining the two elastic bridge members of the distraction plate so as to make them less parallel than their unconstrained state.

## Patentansprüche

1. Vorrichtung zum Bereitstellen von Kompression in einem Körper, wobei die Vorrichtung Folgendes umfasst:
eine Kompressionsplatte (5), die an einer Abgabevorrichtung (200) angebracht ist, wobei die Kompressionsplatte (5) eine Mittellinie aufweist, die erste und zweite gegenüberliegende Bereiche (10) aufweist, die durch mindestens ein elastisches Brückenelement (15) miteinander verbunden sind, **dadurch gekennzeichnet, dass**
das mindestens eine elastische Brückenelement (15) eine Mitte beinhaltet, wobei das mindestens eine elastische Brückenelement (15) eine nicht lineare Konfiguration aufweist und die Mitte des mindestens einen elastischen Brückenelements (15) relativ zur Mittellinie nach außen gebogen ist, wenn sich das mindestens eine elastische Brückenelement (15) in seinem nicht vorgespannten Zustand befindet, aber bei Krafteinwirkung auf das mindestens eine elastische Brückenelement (15) elastisch zu einer lineareren Konfiguration verformt werden kann, und mindestens eine Öffnung (20), die in jedem der ersten und zweiten gegenüberliegenden Bereiche (10) gebildet ist, wobei die mindestens eine Öffnung (20) in jedem der ersten und zweiten gegenüberliegenden Bereiche (10) konfiguriert ist, um ein Befestigungselement (120) aufzunehmen;
derart, dass die Öffnungen (20) in den ersten und zweiten gegenüberliegenden Bereichen (10) durch einen ersten Abstand getrennt sind, wenn sich das mindestens eine elastische Brückenelement (15) in seiner nicht linearen Konfiguration befindet, und die Öffnungen (20) in den ersten und zweiten gegenüberliegenden Bereichen (10) durch einen zweiten Abstand getrennt sind, wenn sich das mindestens eine elastische Brückenelement (15) in seiner lineareren Konfiguration befindet, und wobei ferner der zweite Abstand größer als der erste Abstand ist; und
die Abgabevorrichtung die beiden elastischen Brückenelemente der Kompressionsplatte elastisch spannt, um sie zumindest paralleler als in ihrem uneingeschränkten Zustand auszurichten.

2. Vorrichtung nach Anspruch 1, wobei die Kompressionsplatte (5) zwei elastische Brückenelemente (15) umfasst, wobei die zwei elastischen Brückenelemente (15) auf beiden Seiten der Mittellinie angeordnet sind, und wobei die zwei elastischen Brückenelemente (15) ferner relativ zur Mittellinie nach außen gebogen sind, wenn sich die zwei elastischen Brückenelemente (15) in ihrem nicht vorgespannten Zustand befinden.

3. Vorrichtung nach Anspruch 1, wobei das mindestens eine elastische Brückenelement (15) in seinem nicht vorgespannten Zustand eine "S"-Konfiguration aufweist.

4. Vorrichtung nach Anspruch 1, wobei jeder der ersten und zweiten gegenüberliegenden Bereiche (10) eine Vielzahl von Öffnungen (20) umfasst.

5. Vorrichtung nach einem der vorhergehenden Ansprüche, wobei die Kompressionsplatte (5) aus einem Formgedächtnismaterial gebildet ist, wobei das Formgedächtnismaterial Nitinol umfasst.

6. Vorrichtung nach Anspruch 1, wobei ein Abschnitt der Kompressionsplatte (5) plastisch verformbar ist, wobei mindestens einer der ersten und zweiten gegenüberliegenden Bereiche (10) plastisch verformbar ist.

7. Vorrichtung nach Anspruch 6, wobei der Abschnitt aus vollständig geglühtem Nitinol oder martensitischem Nitinol mit einer Austenitstarttemperatur besteht, die höher als die Körpertemperatur ist, wobei das mindestens eine Brückenelement (15) aus Austenit gebildet ist, aber spannungsinduzierten Martensit bilden kann.

8. Vorrichtung nach einem der vorhergehenden Ansprüche, wobei die linearere Konfiguration im Wesentlichen gerade ist.

9. Vorrichtung nach Anspruch 1, wobei die Kompressionsplatte (5) an der Abgabevorrichtung (200) angebracht ist, die verwendet werden kann, um das mindestens eine elastische Brückenelement (15) von seiner nicht linearen Konfiguration in seine linearere Konfiguration zu rekonfigurieren, wobei die Abgabevorrichtung (200) vom Benutzer einstellbar ist, um das Ausmaß einzustellen, in dem das mindestens eine Brückenelement (15) rekonfiguriert wird.

10. Sterilisiertes Kit, umfassend eine Vorrichtung nach Anspruch 9, wobei die Kompressionsplatte (5) und die Abgabevorrichtung (200) als sterilisiertes Kit gebündelt sind, wobei die Kompressionsplatte (5) und die Abgabevorrichtung (200) als das sterilisierte Kit gebündelt sind, während die Abgabevorrichtung (200) das mindestens eine elastische Brückenelement (15) in seiner lineareren Konfiguration hält.

11. Vorrichtung nach Anspruch 1, ferner umfassend mindestens ein Befestigungselement (120), das in einer Öffnung angeordnet ist, wobei das mindestens eine Befestigungselement (120) optional eine Befestigungsschraube umfasst, und wobei die mindestens eine Öffnung ferner ein Schraubgewinde umfasst, und wobei die Befestigungsschraube (120) ferner einen Gewindekopf (130) zum Eingreifen in das Schraubgewinde umfasst.

12. Vorrichtung nach einem der vorhergehenden Ansprüche, ferner umfassend einen dritten gegenüberliegenden Bereich (10), der durch mindestens ein zusätzliches elastisches Brückenelement (15) mit mindestens einem der ersten und zweiten gegenüberliegenden Bereiche (10) verbunden ist, wobei das mindestens eine zusätzliche elastische Brückenelement (15) eine nicht lineare Konfiguration aufweist, wenn es sich in seinem nicht vorgespannten Zustand befindet, aber bei Krafteinwirkung auf das mindestens eine zusätzliche elastische Brückenelement (15) elastisch zu einer lineareren Konfiguration verformt werden kann, und mindestens eine zusätzliche Öffnung (20), die im dritten gegenüberliegenden Bereich (10) gebildet ist, wobei die mindestens eine zusätzliche Öffnung (20) konfiguriert ist, um ein Befestigungselement (120) aufzunehmen.

13. Vorrichtung nach Anspruch 1, wobei die Kompressionsplatte (5) konfiguriert ist, um ersten und zweiten Knochenfragmenten über einer Frakturlinie Kompression bereitzustellen,
wobei, wenn das mindestens eine elastische Brückenelement (15) elastisch in der lineareren Konfiguration gespannt ist, die Kompressionsplatte (5) gegen den Knochen positioniert ist, sodass eine der Öffnungen (20) über dem ersten Knochenfragment positioniert ist und eine andere der Öffnungen (20) über dem zweiten Knochenfragment positioniert ist, wobei das mindestens eine elastische Brückenelement (15) die Frakturlinie überspannt,
wobei ein Befestigungselement (120) durch eine Öffnung (20) und in das erste Knochenfragment und ein anderes Befestigungselement (120) durch die andere Öffnung (20) und in das zweite Knochenfragment verläuft, und
wobei die Kompressionsplatte (5) eine Kompressionskraft über der Frakturlinie ausübt, wenn die Spannung auf das mindestens eine elastische Brückenelement (15) freigesetzt wird.

14. Vorrichtung nach Anspruch 1, wobei das mindestens eine Brückenelement (15) aus Austenit gebildet, aber spannungsinduzierten Martensit bilden kann;
und wobei ferner mindestens einer der gegenüberliegenden Bereiche (10) aus vollständig geglühtem Nitinol oder martensitischem Nitinol mit einer Austenitstarttemperatur gebildet ist, die höher als die Körpertemperatur ist.

15. Vorrichtung zum Bereitstellen von Distraktion in einem Körper, wobei die Vorrichtung Folgendes umfasst:
eine Distraktionsplatte (505), die erste und zweite gegenüberliegende Bereiche (510) umfasst, die durch mindestens ein elastisches Brückenelement (515) miteinander verbunden sind, wobei das mindestens eine elastische Brückenelement (515) eine lineare Konfiguration aufweist,
wenn es sich in seinem nicht vorgespannten Zustand befindet, aber bei der Krafteinwirkung auf das mindestens eine elastische Brückenelement (515) elastisch in eine weniger lineare Konfiguration verformt werden kann, und mindestens eine Öffnung (520), die in jedem der ersten und zweiten gegenüberliegenden Bereiche (510) gebildet ist,
wobei die mindestens eine Öffnung (520) in jedem der ersten und zweiten gegenüberliegenden Bereiche (510) konfiguriert ist, um ein Befestigungselement aufzunehmen;
derart, dass die Öffnungen (520) in den ersten und zweiten gegenüberliegenden Bereichen (510) durch einen ersten Abstand getrennt sind, wenn sich das mindestens eine elastische Brückenelement (515) in seiner linearen Konfiguration befindet, und die Öffnungen (520) in den ersten und zweiten gegenüberliegenden Bereichen (510) durch einen zweiten Abstand getrennt sind, wenn sich das mindestens eine elastische Brückenelement (515) in seiner weniger linearen Konfiguration befindet, und wobei ferner der erste Abstand größer als der zweite Abstand ist, und
die Distraktionsplatte (505) an einer Abgabevorrichtung (200) angebracht ist, wobei die Abgabevorrichtung elastisch die beiden elastischen Brückenelemente der Distraktionsplatte spannt, um sie weniger parallel als in ihrem uneingeschränkten Zustand auszurichten.

## Revendications

1. Appareil pour fournir une compression au sein d'un corps, ledit appareil comprenant :
une plaque de compression (5) montée sur un dispositif de mise en place (200), la plaque de compression (5) ayant une ligne centrale comprenant des première et deuxième régions opposées (10) raccordées ensemble par au moins un élément de pont élastique (15), **caractérisé en ce que**
ledit au moins un élément de pont élastique (15) inclut un centre, dans lequel ledit au moins un élément de pont élastique (15) a une configuration non linéaire et ledit centre dudit au moins un élément de pont élastique (15) est arqué vers l'extérieur par rapport à ladite ligne centrale lorsque l'au moins un élément de pont élastique (15) est dans son état non sollicité mais est capable d'une déformation élastique en une configuration plus linéaire lors de l'application d'une force audit audit au moins un élément de pont élastique (15), et au moins une ouverture (20) formée dans chacune desdites première et deuxième régions opposées (10), dans lequel ladite au moins une ouverture (20) dans chacune desdites première et deuxième régions opposées (10) est configurée pour recevoir un dispositif de fixation (120) ;
de sorte que lesdites ouvertures (20) dans lesdites première et deuxième régions opposées (10) sont séparées d'une première distance lorsque ledit au moins un élément de pont élastique (15) est dans sa dite configuration non linéaire, et lesdites ouvertures (20) dans lesdites première et deuxième régions opposées (10) sont séparées d'une deuxième distance lorsque ledit au moins un élément de pont élastique (15) est dans sa dite configuration plus linéaire, et en outre dans lequel ladite deuxième distance est supérieure à ladite première distance ; et
le dispositif de mise en place exerce un effort élastique sur les deux éléments de ponts élastiques de la plaque de compression de façon à les rendre au moins plus parallèles que leur état non contraint.

2. Appareil selon la revendication 1, dans lequel ladite plaque de compression (5) comprend deux éléments de ponts élastiques (15), dans lequel lesdits deux éléments de ponts élastiques (15) sont disposés de chaque côté de ladite ligne centrale, et en outre dans lequel lesdits deux éléments de ponts élastiques (15) sont arqués vers l'extérieur par rapport à ladite ligne centrale lorsque lesdits deux éléments de ponts élastiques (15) sont dans leur état non sollicité.

3. Appareil selon la revendication 1, dans lequel ledit au moins un élément de pont élastique (15) a une configuration en « S » dans son état non sollicité.

4. Appareil selon la revendication 1, dans lequel chacune desdites première et deuxième régions opposées (10) comprend une pluralité d'ouvertures (20).

5. Appareil selon l'une quelconque des revendications précédentes, dans lequel ladite plaque de compression (5) est formée à partir d'un matériau à mémoire de forme, dans lequel ledit matériau à mémoire de forme comprend du nitinol.

6. Appareil selon la revendication 1, dans lequel une portion de ladite plaque de compression (5) est déformable plastiquement, dans lequel au moins l'une desdites première et deuxième régions opposées (10) est déformable plastiquement.

7. Appareil selon la revendication 6, dans lequel ladite portion est entièrement en nitinol recuit ou nitinol martensitique avec une température de démarrage d'austénite supérieure à la température du corps, dans lequel ledit au moins un élément de pont (15) est formé à partir d'austénite, mais capable de former une martensite induite par contrainte.

8. Appareil selon l'une quelconque des revendications précédentes, dans lequel ladite configuration plus linéaire est sensiblement droite.

9. Appareil selon la revendication 1, dans lequel ladite plaque de compression (5) est montée sur le dispositif de mise en place (200) qui peut être utilisé pour reconfigurer ledit au moins un élément de pont élastique (15) de sa dite configuration non linéaire à sa dite configuration plus linéaire, dans lequel ledit dispositif de mise en place (200) est réglable par l'utilisateur pour définir dans quelle mesure ledit au moins un élément de pont (15) est reconfiguré.

10. Kit stérilisé comprenant un appareil selon la revendication 9, dans lequel ladite plaque de compression (5) et ledit dispositif de mise en place (200) sont conditionnés sous la forme du kit stérilisé, dans lequel ladite plaque de compression (5) et ledit dispositif de mise en place (200) sont conditionnés sous la forme du kit stérilisé tandis que ledit dispositif de mise en place (200) maintient ledit au moins un élément de pont élastique (15) dans sa dite configuration plus linéaire.

11. Appareil selon la revendication 1, comprenant en outre au moins un dispositif de fixation (120) disposé dans une ouverture, facultativement dans lequel ledit au moins un dispositif de fixation (120) comprend une vis de fixation, et en outre dans lequel ladite au moins une ouverture comprend un filet de vis, et en outre dans lequel ladite vis de fixation (120) comprend une tête filetée (130) pour mettre en prise ledit filet de vis.

12. Appareil selon l'une quelconque des revendications précédentes, comprenant en outre une troisième région opposée (10) raccordée par au moins un élément de pont élastique (15) supplémentaire à au moins une desdites première et deuxième régions opposées (10), dans lequel ledit au moins un élément de pont élastique (15) supplémentaire a une configuration non linéaire lorsqu'il est dans son état non sollicité, mais est capable d'une déformation élastique vers une configuration plus linéaire lors de l'application d'une force audit au moins un élément de pont élastique (15) supplémentaire, et au moins une ouverture (20) supplémentaire formée dans ladite troisième région opposée (10), dans lequel ladite au moins une ouverture (20) supplémentaire est configurée pour recevoir un dispositif de fixation (120).

13. Appareil selon la revendication 1, dans lequel la plaque de compression (5) est configurée pour fournir une compression à des premier et deuxième fragments osseux en travers d'une ligne de fracture,
dans lequel lorsque ledit au moins un élément de pont élastique (15) est soumis à un effort élastique dans ladite configuration plus linéaire, ladite plaque de compression (5) est positionnée contre l'os de sorte que l'une desdites ouvertures (20) est positionnée sur le premier fragment osseux et qu'une autre desdites ouvertures (20) est positionnée sur le deuxième fragment osseux, ledit au moins un élément de pont élastique (15) enjambant la ligne de fracture,
dans lequel un dispositif de fixation (120) passe à travers une ouverture (20) et dans le premier fragment osseux et un autre dispositif de fixation (120) passe à travers l'autre ouverture (20) et dans le deuxième fragment osseux, et
dans lequel ladite plaque de compression (5) applique une force de compression en travers de la ligne de fracture lorsque l'effort sur ledit au moins un élément de pont élastique (15) est relâché.

14. Appareil selon la revendication 1, dans lequel ledit au moins un élément de pont (15) est formé à partir d'austénite, mais capable de former une martensite induite par contrainte ;
et en outre dans lequel au moins l'une desdites régions opposées (10) est formée à partir de nitinol entièrement recuit ou de nitinol martensitique avec une température de démarrage d'austénite supérieure à la température du corps.

15. Appareil pour fournir une distraction au sein d'un corps, ledit appareil comprenant :
une plaque de distraction (505) comprenant des première et deuxième régions opposées (510) raccordées ensemble par au moins un élément de pont élastique (515), dans lequel ledit au moins un élément de pont élastique (515) a une configuration linéaire lorsqu'il est dans son état non sollicité, mais est capable d'une déformation élastique vers une configuration moins linéaire lors de l'application d'une force audit au moins un élément de pont élastique (515), et au moins une ouverture (520) formée dans chacune desdites première et deuxième régions opposées (510),
dans lequel ladite au moins une ouverture (520) dans chacune desdites première et deuxième régions opposées (510) est configurée pour recevoir un dispositif de fixation ;
de sorte que lesdites ouvertures (520) dans lesdites première et deuxième régions opposées (510) sont séparées d'une première distance lorsque ledit au moins un élément de pont élastique (515) est dans sa dite configuration linéaire, et lesdites ouvertures (520) dans lesdites première et deuxième régions opposées (510) sont séparées d'une deuxième distance lorsque ledit au moins un élément de pont élastique (515) est dans sa dite configuration moins linéaire, et en outre dans lequel ladite première distance est supérieure à ladite deuxième distance, et
la plaque de distraction (505) est montée sur un dispositif de mise en place (200), le dispositif de mise en place exerce un effort élastique sur les deux éléments de ponts élastiques de la plaque de distraction afin de les rendre moins parallèles que leur état non contraint.
